# EUROPEAN PATENT APPLICATION

(11) **EP 2 087 902 A1**
(43) Date of publication of application: **12.08.2009**
(21) Application number: 09151504.9
(22) Date of filing: 28.01.2009
(51) Int. Cl.: A61K 36/31, A61K 9/00

(54) **Oral formulations designed to protect the airways, with special reference to inflammatory and neoplastic symptoms**

(30) Priority: 05.02.2008 IT MI2008 172
(71) Applicant: Bios Line S.p.a., 35020 Ponte S. Nicolo (PD) (IT)
(72) Inventor: Tramonti, Paolo, 35100, PADOVA (IT)
(74) Representative: Minoja, Fabrizio

(57) **Abstract**

Disclosed are oral compositions containing the following active ingredients:
- sulphoraphane or other isothiocyanates obtained from Brassicaceae or their precursors, and/or extracts containing them;
- lipoic acid;
- N-acetylcysteine.

## Description

The present invention relates to oral compositions containing, as active ingredients, sulphoraphane or other isothiocyanates obtained from Brassicaceae or their precursors and/or extracts containing them, lipoic acid and N-acetylcysteine.

The compositions according to the invention are useful for the treatment and/or prevention of respiratory disorders with an inflammatory and/or neoplastic basis.

### Prior art

Respiratory failure, a very widespread condition in people of all ages, is not associated with a specific cause, but represents a symptom of various irritant/inflammatory, neoplastic, infectious, metabolic/genetic and traumatic disorders. Smoking and air pollution are obviously among the main causes of respiratory disease and the consequent respiratory failure, characterised by obstructive/inflammatory disorders (bronchitis, emphysema, etc.). Inflammatory symptoms can degenerate to neoplastic forms, although the latter can also arise independently.

The smoking habit and air pollution, especially in cities, are a major cause (or contributory cause) of respiratory problems of various types which limit the efficiency of the bronchopulmonary ventilation capacity. For example, bronchitis, especially in its chronic form, is a slowly developing disease typical of habitual smokers and the inhabitants of towns with a high degree of pollution. It is a serious condition, and by no means negligible, because in the long term it can cause atypical metaplasia and dysplasia of the respiratory epithelium which is usually associated with heart failure.

In any event, both obstructive and neoplastic respiratory disorders are basically characterised by a glandular dysfunction, with excessive secretions and oxidative tissue damage.

Although different therapeutic aids are available which influence the various components of respiratory disorders, such as bronchodilators, mucolytics, antibiotics, non-steroidal anti-inflammatory drugs, corticosteroids, chemotherapy drugs and the like, the need is still felt for products with preventive as well as therapeutic activity, which have no side effects, are preferably of natural origin, and can also be taken as food supplements/nutraceuticals.

The inverse relationship between vegetable consumption and the onset of neoplastic and other disorders is now scientifically recognised. This has led to increased interest in chemoprevention, namely an alternative strategy for controlling the onset of various chronic-degenerative disorders, and a number of nutritional and dietary factors of great interest due to their considerable ability to act as chemopreventive agents have been identified.

Among the natural chemopreventive compounds studied, the isothiocyanates, mainly extracted from Cruciferae (ITC), have aroused the greatest interest, in view of the anticarcinogenic and anti-inflammatory properties demonstrated in experimental models *in vitro* and *in vivo.* These phytoderivatives surprisingly synergise with the action of other compounds, boosting the natural defence systems of the airways and reducing the possibility of onset of respiratory failure due to chronic-degenerative respiratory disease induced by irritant/inflammatory and other processes. They also constitute a good quasi-nutritional treatment designed to reduce the symptoms in the event of existing insufficient respiratory capacity.

### Description of the invention

It has now been found that a combination of isothiocyanates, such as sulphoraphane and derivatives present in Cruciferae/Brassicaceae, lipoic acid and N-acetylcysteine, is particularly effective in combating both the hypersecretion phenomenon, which occurs at the local mucosa, and oxidative stress and the resulting inflammatory cascade, thus preventing the development of respiratory diseases with various etiologies.

The isothiocyanates extracted from Cruciferae (ITC), as already stated, have long been studied in view of their potential chemopreventive and anti-inflammatory properties.

Said isothiocyanates are present in plants in the form of glucosinolates (GL), and are released when the plant tissue is damaged, or as a result of chewing. The conversion of GL to ITC is catalysed by myrosinase (thioglucoside glucohydrolase), an enzyme present in the same plants.

Although over 120 different GLs, the majority of which produce ITC, have been identified in various plants, in practice only a small number of ITCs can be commonly consumed in appreciable amounts by humans. It should be borne in mind that normal cooking processes, by destroying myrosinase, reduce the possibility that glucosinolates will be converted to isothiocyanates, although a limited contribution in this respect is made by the intestinal bacterial flora. For this reason, effective prevention of chronic-degenerative disorders would require a very high dietary intake of plant sources of ITC.

The plasma absorption of these compounds is generally determined indirectly in the urine, exploiting the quantitative reaction with 1,2-benzene dithiol to form 1,3-benzo-dithiol-2-thione.

These clinical biochemical investigations demonstrate the good oral bioavailability of said compounds, for which some form of carrier (phytosomes, co-ground products, liposomes and terclathrates) is not compulsory, but remains optional; however, as these compounds tend to be volatile and have a characteristic odour, for some particular forms of administration it may be useful to use said compounds in microencapsulated, terclathrated or other form. Examples of Cruciferae which are particularly rich in GL and a source of specific ITCs, are mustard and horseradish (allyl-ITC), watercress (phenylethyl-ITC) and broccoli (sulphoraphane).

The anti-neoplastic role played by these compounds is also very important. ITC inhibits the development of the carcinogenesis process by means of multiple mechanisms, including: (i) DNA protection through modulation of the enzymes involved in the metabolism of carcinogenic agents; (ii) reduction of oxidative stress following an increase in cell antioxidant levels and their maintenance; (iii) inhibition of cell proliferation mediated by inhibition or blockage of clonal expansion of metaplastic, dysplastic or neoplastic cells. ITCs also modulate simultaneously a number of target cells which are known to play a crucial part in the carcinogenesis process. Some ITCs also perform their activity towards drug-resistant tumour cells (MDR).

Sulphoraphane (SF) is the isothiocyanate in the ITC class which has been most extensively studied, and also the most promising: oral administration of SF in the rat causes a significant reduction in the incidence of chemically-induced mammary tumours. SF causes significant lengthy protection against oxidative damage in pigmented epithelial cells of the human retina, keratinocytes and mouse leukaemia cells. The molecular bases of the chemopreventive activity of SF are mainly known: the compound not only inhibits the development of the tumour, but also performs a cytotoxic action. Modulation of the carcinogen metabolism, due to inhibition of cytochrome P450-dependent monooxygenase and/or inducement of detoxification enzymes like glutathione (GSH) transferase, is considered to be responsible for the activity of SF against chemically-induced tumours.

N-acetylcysteine (NAC), which has long been used as a mucolytic, possesses antioxidant properties and is a precursor of L-cysteine, an aminoacid essential for cell glutathione (GSH) synthesis. NAC plays a leading part in maintaining adequate GSH levels, thus helping to protect the cells against various toxic agents. It has also been demonstrated that NAC reduces cytoxicity caused by chemotherapy drugs, performs an adjuvant action in the treatment of AIDS, is an effective antidote to paracetamol poisoning, is effective in the prophylaxis of chronic bronchitis, and prevents renal toxicity caused by contrast media.

N-acetylcysteine also protects alpha-antitrypsin, an elastase-inhibiting enzyme, against deactivation induced by hypochlorous acid, a powerful oxidant produced by the enzyme myeloperoxidase in activated phagocytes, and thus performs a powerful cell antioxidant action. The structure of the molecule also allows it to cross the cell membranes easily. In the cell, NAC is deacetylated and releases L-cysteine, which is essential for cell glutathione (GSH) synthesis. NAC therefore plays a crucial part in maintaining adequate GSH levels, helping to protect the cells against toxic agents. NAC is therefore useful to combat oxidative damage of various kinds, such as that attributable to hydrogen peroxide, hypochlorous acid, hydroxy radicals, cigarette condensate, peroxynitrates, superoxide anion, and singlet oxygen species. According to well-established studies, NAC also reduces cytotoxicity caused by antitumoral chemotherapy drugs and, as already described, performs an adjuvant action in the treatment of AIDS. NAC is also a substance able to inhibit the genotoxic processes and carcinogenesis associated with various chemical agents. Finally (according to a study published by Aluigi MG et al, Anticancer Res 2000; 20: 3183-3187), NAC inhibits the invasive process and metastatisation of malignant cells.

A recent study conducted on laboratory animals evaluated the exposure to cigarette smoke, the possible lung damage and the antioxidant properties of NAC. The damage produced by cigarette smoke involved the thickness of the bronchial wall and caused an increasingly inefficient distribution of ventilation. NAC has proved to be able to prevent these alterations to a statistically significant extent.

Alpha-lipoic acid, also called lipoic or thioctic acid, is an essential cofactor for many enzyme complexes, specifically for pyruvate dehydrogenase, an enzyme which plays an essential part in the aerobic metabolism.

Lipoic acid, which is present in animal and plant tissues (liver, heart, kidneys, spinach, potatoes and broccoli), has been used as an anti-hepatotoxic agent and, as a diet supplement, as an antioxidant and anti-free radical agent.

Recent studies have demonstrated that lipoic acid is also important to control glucose and prevent disorders like cataract and stroke. A daily dose of around 50 mg is currently recommended for healthy persons, while doses of over 600 mg/day are recommended for diabetics. Lipoic acid is absorbed almost totally by oral administration (>80%) and is evenly distributed in the tissues, where it promotes GSH synthesis; it prevents vitamin C oxidation mediated by Cu++ ions, and promotes reduction of oxidised vitamins C and E.

Said components of the formulations according to the invention interact synergically, producing advantageous anti-hypersecretory, anti-oxidative, anti-inflammatory and anti-neoplastic effects in the respiratory tract. These actions are particularly useful to combat respiratory disorders mainly deriving from the smoking habit and involuntary inhalation of irritant gases, particulate matter and other environmental pollutants.

The compositions according to the invention may also contain one or more of the following ingredients:
1) water- or fat-soluble vitamins (group B, C, E, etc.);
2) vitamin-like compounds or their carriers (e.g. choline and citicoline);
3) antioxidant compounds of plant origin in pure form or extracts containing them, possibly complexed with hydrophilic or lyophilic carriers, preferably polyphenols of vine, green tea, milk thistle and Ginkgo biloba.
4) carotenes, lutein, lycopene, astaxanthin, zeaxanthin or mixtures thereof;
5) trace elements acting as cofactors of enzymes with antioxidant activity;
6) coenzyme Q10 or reduced glutathione (GSH);
7) substances that contribute sulphated aminoacids (e.g. S-adenosylmethionine or SAMe);
8) cysteine, cystine, methionine or extracts containing them (e.g. millet).

The compositions according to the invention can be conveniently formulated in any form suitable for oral administration, including solid forms such as powders, granulates, capsules, tablets or liquid oral forms, possibly with controlled release (time- and/or pH-dependent).

The ITCs will be present in the formulations according to the invention in the form of a titrated plant extract (minimum 1%) in such quantities as to provide a daily dose of 0.1 to 1000 mg (although doses of between 2 and 20 mg will preferably be used) considered as pure ITCa, NAC 10 to 2400 mg (preferably 400 mg), and lipoic acid 1 to 1500 mg (preferably 100 mg).

The doses of the other ingredients can vary within wide ranges, but will be approximately as follows:
1) vitamins: 10 to 300% of the RDA (preferably 150% RDA);
2) vitamin-like compounds: 1 to 400 mg (preferably 100 mg);
3) antioxidant herbal derivatives: 10 to 1200 mg (preferably 150 mg);
4) carotenoids: 0.1 to 24 mg (preferably 6 mg);
5) trace elements: 10 to 150% of the RDA (preferably 100% RDA);
6) Coenzyme Q10 or GSH: 1 to 1000 mg (preferably 20 mg);
7) S-adenosylmethionine: 10 to 1000 mg (preferably 100 mg);
8) sulphated aminoacids: 10 to 1500 mg (preferably 100 mg).

Some examples of formulations according to the invention are set out below.

### EXAMPLES

**1) TYPE 0 CAPSULES**

| | |
|---|---|
| Broccoli extract | 100 mg |
| NAC | 100 mg |
| Lipoic acid | 200 mg |
| Microcel | 50 mg |
| Vegetable magnesium stearate | 7 mg |
| Aerosil | 10 mg |

**2) FILM-COATED TABLETS**

| | |
|---|---|
| Broccoli extract | 200 mg |
| NAC | 200 mg |
| Lipoic acid | 100 mg |
| Microcel | 440 mg |
| Dicafos | 333 mg |
| Vegetable magnesium stearate | 10 mg |
| Aerosil | 8 mg |
| Explocel | 18 mg |
| Sepifilm | 22,8 mg |
| Shellac | 7 mg |
| Colouring | 0,2 mg |

**3) TWO-LAYER CONTROLLED-RELEASE TABLETS**

| NORMAL-RELEASE LAYER | |
|---|---|
| Broccoli extract | 100 mg |
| NAC | 100 mg |
| Lipoic acid | 50 mg |
| Dicafos | 304 mg |
| Aerosil | 3 mg |
| Vegetable magnesium stearate | 6 mg |
| Colouring | 1 mg |

| SLOW-RELEASE LAYER | |
|---|---|
| Broccoli extract | 200 mg |
| NAC | 100 mg |
| Lipoic acid | 50 mg |
| Metholose | 80 mg |
| Aerosil | 2 mg |
| Vegetable magnesium stearate | 3 mg |
| Microcel | 80 mg |
| Dicafos | 164 mg |

| **4) SACHETS** | |
|---|---|
| Terclathrated broccoli extract | 100 mg |
| NAC | 100 mg |
| Lipoic acid | 100 mg |
| Fructose | 2000 mg |
| Aerosil | 15 mg |
| Orange flavouring | 180 mg |
| Mandarin flavouring | 40 mg |
| Citric acid | 220 mg |
| Acesulfame k | 25 mg |
| E102 | 2 mg |

**5) ORODISPERSIBLE FORMULATION**

| | |
|---|---|
| Micronised broccoli extract | 150 mg |
| NAC | 200 mg |
| Lipoic acid | 50 mg |
| Sorbitol | 160 mg |
| Orange flavouring | 20 mg |
| Mandarin flavouring | 5 mg |
| Acesulfame k | 2 mg |
| Aerosil | 5 mg |
| Fructose | 1566 mg |

**6) GUM DISC**

| | |
|---|---|
| Broccoli extract phytosome | 300 mg |
| NAC | 50 mg |
| Lipoic acid | 10 mg |
| Gum base | 800 mg |
| Aspartame | 2 mg |
| Menthol | 2 mg |
| Acesulfame | 1 mg |
| Levilite | 20 mg |
| Talc F.U. | 20 mg |
| Vegetable magnesium stearate | 18 mg |
| Shellac | 12 mg |
| Xylitol | 250 mg |
| Gum arabic | 6 mg |
| Titanium dioxide | 6 mg |
| Carnauba wax | 0,2 mg |

**7) SACHETS**

| | |
|---|---|
| Broccoli extract (co-ground) | 100 mg |
| NAC | 200 mg |
| Lipoic acid | 100 mg |
| Vitamin C | 180 mg |
| Vitamin E | 30 mg |
| Selenium | 0,01 mg |
| Citicoline | 100 mg |
| CoQ10 | 10 mg |
| Fructose | 2000 mg |
| Aerosil | 15 mg |
| Orange flavouring | 180 mg |
| Mandarin flavouring | 40 mg |
| Citric acid | 220 mg |
| Acesulfame k | 25 mg |
| E102 | 2 mg |

## Claims

1. Oral compositions containing the following active ingredients:
- sulphoraphane or other isothiocyanates obtained from Brassicaceae or their precursors, and/or extracts containing them;
- lipoic acid;
- N-acetylcysteine.

2. Compositions as claimed in claim 1, containing broccoli extract as a source of sulphoraphane or isothiocyanates.

3. Compositions as claimed in claim 1 or 2, also containing one or more ingredients selected from:
- water- or fat-soluble vitamins;
- vitamin-like compounds;
- antioxidant compounds of plant origin in pure form or extracts containing them, possibly complexed with hydrophilic or lyophilic carriers;
- carotenes, lutein, lycopene, astaxanthin, zeaxanthin;
- trace elements acting as cofactors of enzymes with antioxidant activity;
- coenzyme Q10 or reduced glutathione (GSH);
- cysteine, cystine, methionine, and their precursors or extracts containing them.

4. Compositions as claimed in claim 3, wherein the vitamin-like compounds are choline or citicoline.

5. Compositions as claimed in claim 3 or 4, wherein the antioxidant compounds of plant origin are selected from polyphenols of vine, green tea, milk thistle and Ginkgo biloba.

6. Compositions as claimed in any of the preceding claims, in the form of capsules, tablets, controlled-release forms, sachets, orodispersible granulate and chewing gum.

7. Use of a combination of N-acetylcysteine, lipoic acid and sulphoraphane or other isothiocyanates obtained from Brassicaceae or their precursors and/or extracts containing them to prepare chemopreventive, antioxidant and airway anti-inflammatory agents.
